# EUROPEAN PATENT SPECIFICATION

(11) **EP 2 830 605 B1**
(45) Date of publication and mention of the grant of the patent: **27.09.2023**
(21) Application number: 14746905.0
(22) Date of filing: 07.01.2014
(51) Int. Cl.: A61K 31/137, A61K 31/135, A61K 31/167, A61P 11/00, A61P 11/02, A61P 11/12

(54) **A COMBINATION MEDICAMENT COMPRISING PHENYLEPHRINE AND PARACETAMOL**
KOMBINATIONSMEDIKAMENT MIT PHENYLEPHRIN UND PARACETAMOL
COMBINAISON MÉDICAMENTEUSE COMPRENANT LA PHÉNYLÉPHRINE ET LE PARACÉTAMOL

(30) Priority: 04.02.2013 NZ 60665913; 02.05.2013 NZ 61013213; 02.08.2013 NZ 61391813; 20.11.2013 NZ 61802713
(43) Date of publication of application: 04.02.2015
(73) Proprietor: AFT Pharmaceuticals Limited, Auckland 0622 (NZ)
(72) Inventor: ATKINSON, Hartley Campbell, Takapuna Auckland 0622 (NZ)
(74) Representative: Arnold & Siedsma
(86) International application number: PCT/NZ2014/000001
(87) International publication number: WO 2014/120021

(56) References cited:
- WO-A1-2004/066978
- WO-A1-2012/090218
- WO-A2-2007/125501
- US-A- 4 260 600
- US-A1- 2007 254 027
- US-A1- 2009 230 013
- US-A1- 2010 136 107
- US-A1- 2011 104 272
- COLDREX P.E: 'Phenylephrine Sinus Tablets, Panadol Sinus Relief PE Tablets, Panadol Cold & Flu Max+Decongestant Tablets' NEW ZEALAND DATASHEET. 13 April 2012, XP055154372 Retrieved from the Internet: <URL:http://www.medsafe.goyt.87/profs/datas heet/c/Coldrextab.pdf> [retrieved on 2013-10-03]
- 'Trial from ANZCTR.' AUSTRALIAN NEW ZEALAND CLINICAL TRIALS REGISTRY. 15 August 2012, XP055154379 Retrieved from the Internet: <URL:https://www.anzctr.org.au/Trial/Regisi ration/TrialReview.aspx?id=362877> [retrieved on 2013-10-02]
- ATKINSON HC ET AL.: 'Increased Phenylephrine Plasma Levels with Administration of Acetaminophen' THE NEW ENGLAND JOURNAL OF MEDICINE vol. 370, no. 12, 20 March 2014, pages 1171 - 1172, XP055154380

## Description

The invention relates to a medicament for treating upper respiratory mucosal congestion in tablet, capsule, powder or liquid form, comprising paracetamol and phenylephrine hydrochloride as active ingredients.

### BACKGROUND

Upper respiratory mucosal congestion caused by infections such as the common cold and influenza can cause a number of unpleasant symptoms. These include congestion of nasal passages, excessive sinus secretions, headache, muscle ache, fever and malaise. It is an object of a preferred form of the invention to go at least some way towards providing a paracetamol + phenylephrine hydrochloride combination which relieves at least some of the above symptoms for at least some people.

### THE PRIOR ART

Paracetamol is a known analgesic available without prescription and typically taken in doses of 650 mg or 1,000 mg, administered as 2 tablets of 500 mg or 325 mg each. Phenylephrine hydrochloride is a known decongestant and is typically taken in doses of 10 mg, administered as one tablet when given alone or as 2 tablets of 5 mg each when given in combination with other agents such as paracetamol.

Combinations containing paracetamol and phenylephrine hydrochloride are known. For example *Codral*^{™} *PE Cold* & *Flu* + *Cough* has 500 mg paracetamol + 5 mg phenylephrine hydrochloride + 10 mg Dextromethorphan Hydrobromide in capsule form. The product is to be taken 2 capsules at a time to give an adult dose of 1,000 mg paracetamol + 10 mg phenylephrine hydrochloride + 20 mg Dextromethorphan Hydrobromide every 4-6 hours.

Another example is *Lemsip*^{™} *Cold* & *Flu* which is available in capsules, each having 25 mg caffeine + 500 mg paracetamol + 6.1 mg phenylephrine hydrochloride (equivalent to 5 mg phenylephrine free base). The product is to be taken 2 capsules at a time to give an adult dose of twice these amounts every 4-6 hours.

A further example is *Panadol^{™} PE Sinus Relief* which is available in caplets each having 500 mg paracetamol + 5 mg phenylephrine hydrochloride. For an adult dose 2 caplets are taken every 4-6 hours.

A further example known in some Asian countries is *No Drowse Decolgen*^{™} which comes in tablets each having 500 mg paracetamol + 10 mg phenylephrine hydrochloride. For an adult dose 1 tablet is taken every 6 hours.

As indicated above, a normal adult dose of phenylephrine hydrochloride is 10 mg delivered in 2 dosage units such as capsules or tablets (eg caplets). A dose of 10 mg phenylephrine hydrochloride provides 8.1 mg of phenylephrine free base. However it is known to administer higher doses such as 12.2 mg phenylephrine hydrochloride to give the equivalent of 10 mg of the free base. The generally accepted dose is therefore an amount sufficient to give the equivalent of 8.1 mg to 10 mg of the free base. The recommended frequency of dosing is 3-4 times daily (*Martindale 28^{th} Edition*) or every 4 hours (*Drug Tx, 4^{th} Edition*).

Patent specification WO 2009/012590 (Kingsway) pages 21 and 29 incorporates a reference to tablets having 50mg Ibuprofen + 80 mg paracetamol + 5 mg phenylephrine. However there is no disclosure as to whether this is postulated for use with adults or children or whether it is for use 2 tablets at a time to deliver 10 mg phenylephrine.

US2011/104272A1 discloses combinations for use in the treatment of upper respiratory mucosal congestion comprising *inter alia* (a) phenylephrine and (b) paracetamol. The phenylephrine is present at an amount chosen from 5 mg, 7.5 mg, 10 mg, 12 mg, 15 mg, 20 mg, 22.5 mg, 25 mg, 30 mg, 32 mg, 34 mg, 35 mg, 37 mg, 40 mg, 50 mg or 60 mg or higher. The paracetamol is present at an amount chosen from about 150 mg, 175mg, 200mg, 225mg, 250mg, 275mg, 300mg, 325mg, 350mg, 400mg, 425mg, 450mg, 500mg, 525mg, 530mg, 535mg, 540mg, 550mg, 600mg, 650mg or about 700mg.

WO2007/125501A2 discloses liquid combinations for use in the treatment of upper respiratorz mucosal congestion comprising *inter alia* (a) 5 mg of phenylephrine and (b) paracetamol in the ranges from about 150 mg to about 1,000 mg, or from about 200 mg to about 750 mg, or from about 500 mg to about 650 mg. Example 1 thereof discloses liquid compositions comprising 1.78% w/w of paracetamol and 0.0033% w/w phenylephrine. The liquid oral composition is administered to mammals in total dosage volumes, per dose, from about 5 mL to about 50 mL, or alternatively from about 10 mL to about 30 mL.

The Datasheet for *Coldrex^{™}* PE Phenylephrine Sinus, *Coldrex^{™}* PE Phenylephrine Congestion Clear, *Panadol^{™}* Sinus Pain & Congestion Relief and for *Panadol^{™}* Cold & Flu Max + Decongestant discloses tablets comprising 500 mg paracetamol and 5 mg phenylephrine hydrochloride.

While it was known to dose 10 mg of phenylephrine in combination with 1,000 mg paracetamol it was not previously known that paracetamol enhances absorption of phenylephrine hydrochloride to the extent that the dose of phenylephrine can be substantially reduced. This important discovery enables at least many patients to take substantially lower doses of phenylephrine hydrochloride without compromising treatment, and without the same risk of adverse side effects applicable with significantly higher doses.

### SUMMARY OF THE INVENTION

According to a first aspect of the invention there is provided a combination medicament for use in the treatment of upper respiratory mucosal congestion in tablet, capsule, powder or liquid form, characterised in that the medicament has the phenylephrine hydrochloride and paracetamol in proportions suitable for, and the medicament is for, providing an adult with:
a) 4 mg - 7.5 mg phenylephrine hydrochloride, in combination with 950 mg - 1 ,000 mg paracetamol; or
b) 5 mg - 7.5 mg phenylephrine hydrochloride, in combination with 600 mg - 700 mg paracetamol.

Optionally the combination medicament for use is in proportions suitable for, and is for, providing an adult with a combination of 5 mg to 6.5 mg phenylephrine hydrochloride and 950 mg - 1,000 mg paracetamol.

Optionally the combination medicament for use is in proportions suitable for, and is for, providing an adult with a combination of 5 mg to 6.2 mg phenylephrine hydrochloride and 950 mg - 1,000 mg paracetamol.

Optionally the combination medicament for use is in proportions suitable for, and is for, providing an adult with a combination of 5 mg phenylephrine hydrochloride and 950 mg - 1,000 mg paracetamol.

Optionally the combination medicament for use is in proportions suitable for, and is for, providing an adult with a combination of 5.8 mg to 7.1 mg phenylephrine hydrochloride and 625 mg - 675 mg paracetamol. Optionally the medicament is in proportions suitable for, and is for, providing an adult with a combination of 5.8 mg phenylephrine hydrochlorideand 625 mg - 675 mg paracetamol.

Optionally the combination medicament for use of part a) or part b) is produced in dosage units containing both named active ingredients sufficient for two dosage units to provide the doses mentioned in part a) or b).

Optionally the combination medicament for use is produced in dosage units containing both named active ingredients sufficient for two dosage units to provide the doses as mentioned hereinabove respectively.

### DRAWINGS

- **Figure 1**: is a graph illustrating increased absorption of phenylephrine hydrochloride when taken in combination with paracetamol;
- **Figure 2**: is a log-linear graph for the data graphed in figure 1;
- **Figure 3**: is a graph further illustrating increased absorption of phenylephrine hydrochloride when taken in combination with paracetamol;
- **Figure 4**: is a log-linear graph for the data graphed in figure 3; and
- **Figure 5**: is a graph for clinical data indicating that 5 mg phenylephrine hydrochloride plus 1,000 mg paracetamol is comparable to 10 mg phenylephrine hydrochloride alone.

### DETAILED DESCRIPTION

Some preferred forms of the invention will now be described by way of example.

The invention is embodied by an oral combination medication in tablet, capsule, powder or liquid form having the analgesic paracetamol and the decongestant phenylephrine hydrochloride as key ingredients.

The medicament is for treating upper respiratory mucosal congestion in adults, for example the type often caused by the common cold. More specifically, the medication may be in the form of tablets or capsules each having:
- 500 mg paracetamol + 2.5 mg phenylephrine hydrochloride; or
- 325 mg paracetamol + 3 mg phenylephrine hydrochloride.

The tablets are to be taken by an adult two at time, every 4-6 hours or on a qid basis, to deliver the following amounts at each dosage event:
- 1,000 mg paracetamol + 5 mg phenylephrine hydrochloride; or
- 650 mg paracetamol + 6 mg phenylephrine hydrochloride.

In alternative embodiments the total dosage amounts referred to in the immediately preceding paragraph are formulated as a single tablet or capsule to be taken by an adult, one per dosage event, every 4-6 hours or on a qid basis, the tablet or capsule having:
- 500 mg paracetamol + 6.25 mg phenylephrine hydrochloride.

It has been surprisingly found that when paracetamol is co-administered with low doses of phenylephrine hydrochloride, the absorption of phenylephrine hydrochloride into the bloodstream is synergistically and significantly increased. While the mechanism behind this is not yet understood, it is believed that the improved absorption may be due to metabolic competition between paracetamol and phenylephrine hydrochloride at the stomach and small intestine. Ordinarily when phenylephrine is taken a reasonable amount is lost to metabolic processes at the stomach and small intestine (first pass metabolism). It is believed that the competition reduces this, to enable more phenylephrine to make it into the bloodstream. It has been determined that with a 5 mg phenylephrine hydrochloride + 1,000 mg paracetamol dose at least many adult bodies absorb about as much therapeutically relevant phenylephrine hydrochloride as for 10 mg phenylephrine hydrochloride alone.

At lower doses of paracetamol such as 500 mg it has been determined that there is still a significant effect, but it is reduced. It has been found that with a 6.25mg phenylephrine hydrochloride + 500 mg paracetamol dose an adult body absorbs about as much therapeutically relevant phenylephrine hydrochloride as for a dose of 10 mg phenylephrine hydrochloride alone.

The invention enables the formulation of a product with less phenylephrine hydrochloride without loss of therapeutic efficacy. It is advantageous because it means patients are less exposed to the risk of adverse side effects. For phenylephrine hydrochloride these include aggravation of underlying hypertension, aggravation of underlying prostatic hyperplasia, rebound hyperemia, greater risk of seizures, upset stomach, abdominal cramping and vomiting. This is important as it has been shown that there is a marked increase in adverse events with increasing dose of phenylephrine hydrochloride¹.
¹ Cohen BM (1972) Clinical and Physiologic Significance of Drug-Induced Changes in Nasal Flow/Resistance. Eur J Clin Pharmacol, 5: 81-86.

| **Dose of Phenylephrine HCL** | **Adverse Event Rate** |
|---|---|
| 10mg | 12.5% |
| 15mg | 43.8% |
| 25mg | 81.3% |

As the applicant has discovered that dosing phenylephrine in combination with paracetamol surprisingly causes an effective increase in the amount of phenylephrine in the bloodstream, a 10 mg dose of phenylephrine in combination is expected to give adverse side effects commensurate with a significantly higher dose. It is estimated that a dose of 10 mg phenylephrine when given with 500-1,000 mg paracetamol is similar to a dose of 16-20 mg phenylephrine hydrochloride alone. As indicated in the table above, phenylephrine hydrochloride at this dose level has an adverse event rate of somewhere between 43.8% - 81.3% compared with 12.5% for 10 mg. The discovery therefore enables phenylephrine/paracetamol combination formulations to be prepared with a reduced risk of adverse side effects without adversely compromising the therapy provided by the phenylephrine.

In preferred forms the tablets or capsules include non-active ingredients, for example binders, colouring agents, film coatings, etc, as appropriate. Examples of common non- active ingredients for the preparation of tablets include maize starch, pre-gelatinised starch, microcrystalline cellulose, micronized stearic acid, magnesium stearate, sodium metabisulphite, disodium edetate and purified water. These, and methods for working them into a tablet or capsule, are well known to a person or team with normal skills in the art.

When the medicament is in the form of tablets they are preferably presented as part of a pack, for example a blister pack. The pack may have an even number of tablets and instructions to take 2 of them up to 4 times a day, or 2 tablets no more than every 4-6 hourly. In some embodiments the tablets may be packaged loose in a bottle with similar instructions.

In a further embodiment the combination medicament for use comprises a combination syrup for administration to patients with difficulty swallowing tablets or capsules. Methods for the production of syrups are well known to those skilled in the art and can be readily employed. The syrup is contained in a bottle, vial or like container and is prepared in a manner suitable for delivering about 4 mg to about 7.5 mg (most preferably about 5 mg) phenylephrine hydrochloride and about 650 mg to about 1000 mg (most preferably about 650 mg or about 1,000 mg) paracetamol per dosage event, up to 4 times daily.

### Example 1

Tablets for dosing to an adult (2 tablets at each dosing event) may be formed according to the table below (the weight amounts are expressed on a per tablet basis).

### 500 mg Paracetamol + 2.5 mg Phenylephrine Hydrochloride

| **Component** | **Quantity/tablet (mg)** |
|---|---|
| **Dry-mixing** | |
| Paracetamol | 500.00 mg |
| Phenylephrine Hydrochloride | 2.50 mg |
| Pregelatinised Starch | 20.832 mg |

| **Granulation** | |
|---|---|
| Maize starch | 34.00 mg |
| Sodium Metabisulphite | 0.585 mg |
| Disodium edetate | 0.0571 mg |
| Colour Quinoline Yellow Supra | 0.0285 mg |
| Purified water<*> | q.s. |

| **Lubrication** | |
|---|---|
| Microcrystalline cellulose (Microcel pH 102) | 20.00 mg |
| Magnesium Stearate | 5.00 mg |
| Stearic acid (micronised) | 2.00 mg |

| | |
|---|---|
| * Purified water is not present in the finished product. | |

### Example 2

Tablets for dosing to an adult, 2 tablets at each dosing event, may be formed according to the table below (the weight amounts are expressed on a per tablet basis).

### 500 mg Paracetamol + 3 mg Phenylephrine Hydrochloride

| **Component** | **Quantity/tablet (mg)** |
|---|---|
| | |

| **Dry-mixing** | |
|---|---|
| Paracetamol | 500.00 mg |
| Phenylephrine Hydrochloride | 3.00 mg |
| Pregelatinised Starch | 20.332 mg |

| **Granulation** | |
|---|---|
| Maize starch | 34.00 mg |
| Sodium Metabisulphite | 0.585 mg |
| Disodium edetate | 0.0571 mg |
| Colour Quinoline Yellow Supra | 0.0285 mg |
| Purified water<*> | q.s. |

| **Lubrication** | |
|---|---|
| Microcrystalline cellulose (Microcel pH 102) | 20.00 mg |
| Magnesium Stearate | 5.00 mg |
| Stearic acid (micronised) | 2.00 mg |

| | |
|---|---|
| * Purified water is not present in the finished product. | |

### Example 3

Tablets for dosing to an adult, 2 tablets at each dosing event, may be formed according to the table below (the weight amounts are expressed on a per tablet basis).

### 325 mg Paracetamol + 3 mg Phenylephrine Hydrochloride

| **Component** | **Quantity/tablet (mg)** |
|---|---|
| **Dry-mixing** | |
| Paracetamol | 325 mg |
| Phenylephrine Hydrochloride | 3.00 mg |
| Pregelatinised Starch | 13.47 mg |

| **Granulation** | |
|---|---|
| Maize starch | 22. mg |
| Sodium Metabisulphite | 0.38 mg |
| Disodium edetate | 0.0571 mg |
| Colour Quinoline Yellow Supra | 0.0285 mg |
| Purified water<*> | q.s. |

| **Lubrication** | |
|---|---|
| Microcrystalline cellulose (Microcel pH 102) | 13.00 mg |
| Magnesium Stearate | 3.25 mg |
| Stearic acid (micronised) | 1.30 mg |

| | |
|---|---|
| * Purified water is not present in the finished product. | |

### Example 4

Tablets for dosing to an adult (1 tablet at each dosing event), may be formed according to the table below (the weight amounts are expressed on a per tablet basis).

| **Component** | **Quantity/tablet (mg)** |
|---|---|
| **Dry-mixing** | |
| Paracetamol | 500.00 mg |
| Phenylephrine Hydrochloride | 6.25 mg |
| Pregelatinised Starch | 17.082 mg |
| | |

| **Granulation** | |
|---|---|
| Maize starch | 34.00 mg |
| Sodium Metabisulphite | 0.585 mg |
| Disodium edetate | 0.0571 mg |
| Colour Quinoline Yellow Supra | 0.0285 mg |
| Purified water<*> | q.s. |

| **Lubrication** | |
|---|---|
| Microcrystalline cellulose (Microcel pH 102) | 20.00 mg |
| Magnesium Stearate | 5.00 mg |
| Stearic acid (micronised) | 2.00 mg |

| | |
|---|---|
| * Purified water is not present in the finished product. | |

It will be noted that for Examples 3 & 4 the amount of paracetamol is less than for Examples 1 and 2. To account for this the amount of phenylephrine hydrochloride is higher.

### CLINICAL TRIALS

A crossover study was run with 28 adult human subjects to compare the absorption of phenylephrine when taken as the only active ingredient with the absorption of phenylephrine hydrochloride when taken in combination with paracetamol. Test subjects were given:
- 10 mg phenylephrine hydrochloride in tablet form (PE HCI alone); or
- 10 mg phenylephrine hydrochloride + 1,000 mg paracetamol + 300 mg ibuprofen in tablet form {*Maxigesic*^{™} PE).

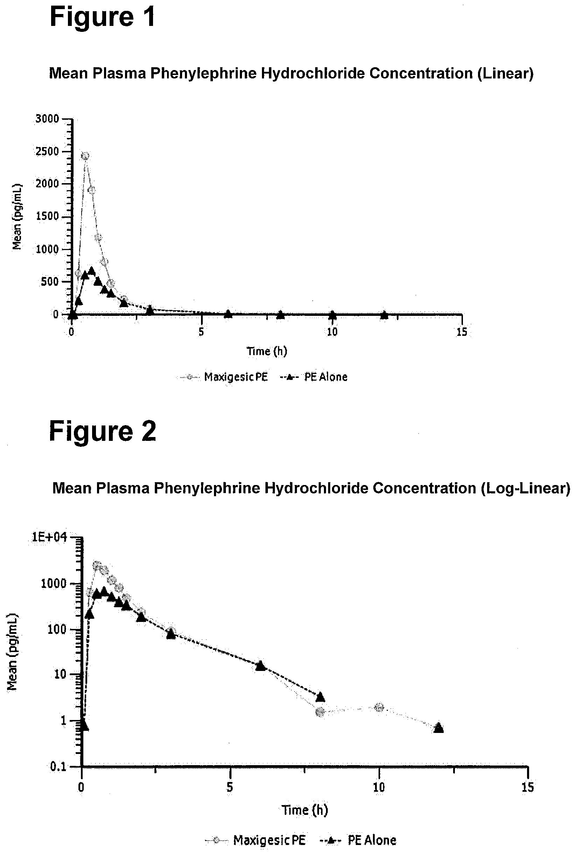

Following ingestion, the amount of phenylephrine in the blood plasma was monitored and the mean results recorded. These are shown graphically at Figure 1 , which indicates that phenylephrine is significantly more available for therapeutic use when taken in combination with paracetamol.

Graphing the results on a log-linear basis as shown at Figure 2 indicates that blood concentrations reduce in parallel, at least between 2 and 6 hours, indicating that the half- life was similar in each case and that the difference in exposure is due to phenylephrine hydrochloride having higher systematic availability when in the presence of paracetamol. This is consistent with a decrease in first pass metabolism of phenylephrine hydrochloride and an increased availability of phenylephrine in the bloodstream.

The study also involved non-compartmental pharmacokinetic analysis and bioequivalence assessment using Kinetica software. Mean (SD) results and 90% confidence intervals are summarized in the table below. The results are indicative of increased bioavailability for phenylephrine hydrochloride when given in combination with paracetamol (Table 1).

**Table 1**

| **Bioequivalence Results** | | | | |
|---|---|---|---|---|
| | **Tmax* (h)** | **Cmax (pg/mL)** | **AUC0n (h*pg/mL)** | **AUCtot (h*pg/mL)** |
| Mean | | | | |
| 10 mg phenylephrine hydrochloride + 1000 mg paracetamol + 300 mg ibuprofen (n=28) | 0.6 | 3220.1 | 2219.8 | 2311.4 |
| 10 mg phenylephrine hydrochloride | 0.7 | 873.8 | 1019.7 | 1104.6 |
| 90% confidence intervals | | | | |
| *Maxigesic^{™}* PE/PE | NC | 260.5-423.7 | 188.6-242.7 | 179.2-233.0 |

While Maxigesic^{™} PE includes ibuprofen, it is believed, on the basis of various clinical trials², that the efficacy of phenylephrine hydrochloride is not improved by ibuprofen. In this regard a second cross-over study was run with 30 adult human subjects to compare the absorption of phenylephrine when taken with different doses of paracetamol, without ibuprofen. Test subjects were given:
- 10 mg phenylephrine hydrochloride in tablet form (*Sudafed*^{™} PE Nasal Decongestant) together with 500 mg paracetamol in tablet from (*Panadof*); or
- 10 mg phenylephrine hydrochloride and 1 ,000 mg paracetamol given in tablet form as two tablets of 5 mg phenylephrine hydrochloride and 500 mg paracetamol (*Sudafed*^{™} PE Sinus and Pain Relief).
²http://www.accessdata.fda.gov/drugsatfda_docs/nda/20 1/022113Orig1 s000ClinPharmR.pdf.

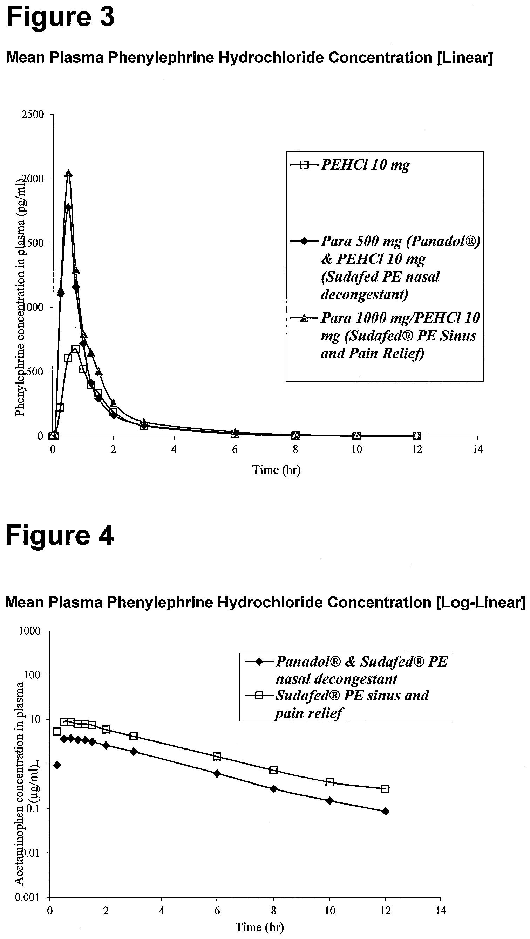

Following ingestion, the amount of phenylephrine in the blood plasma was monitored and mean results recorded. These are shown in the graph at Figure 3 together with the original phenylephrine hydrochloride alone data from the first experiment. These indicate that phenylephrine is significantly more available for therapeutic use when taken in combination with a range of paracetamol doses i.e. 500-1,000 mg. Graphing the results as at Figure 4 on a log-linear basis indicates that the blood concentrations reduce in parallel independent of the paracetamol dose.

Comparing the pharmacokinetic data for the two treatment groups shown below (Table 2) indicates that the dose of paracetamol has an effect on the interaction, with the ratio being around 80% for the lower dose of paracetamol (500 mg) when compared with the higher dose (1 ,000mg). This is believed to be due to the paracetamol competing with phenylephrine for metabolism as it is absorbed across the gut wall. In other words, when there is less paracetamol such as 500 mg there is a lessor interaction than seen for the higher dose of 1 ,000 mg. However it is surprising that some low doses of paracetamol interact as above.

**Table 2**

| **Pharmacokinetic Results of Patients from Second Study** | | | | |
|---|---|---|---|---|
| | **Tmax* (h)** | **Cmax (pg/mL)** | **AUC0n (h*pg/mL)** | **AUCtot (h*pg/mL)** |
| **Mean (SD)** | | | | |
| 10 mg phenylephrine hydrochloride (n=30) + 1000 mg paracetamol | 0.5 | 2545.8 | 2088.5 | 2192.0 |
| 10 mg phenylephrine hydrochloride + 500 mg paracetamol (n = 30) | 0.5 | 2077.2 | 1673.5 | 1779.8 |
| **Ratio** | **ND** | **81.5%** | **80.1 %** | **81.1 %** |

A number of the patients from the first study were also enrolled into the second study [N=14]. The phenylephrine pharmacokinetic results were very similar between the two studies for phenylephrine as shown in the table below (Table 3).

**Table 3**

| **Pharmacokinetic Results of Patients from the First and Second Studies** | | | | |
|---|---|---|---|---|
| | **Tmax* (h)** | **Cmax (pg/mL)** | **AUC0n (h*pg/mL)** | **AUCtot (h*pg/mL)** |
| **Mean (SD)** | | | | |
| 10 mg phenylephrine hydrochloride + 1,000 mg paracetamol (n = 14) | 0.5 | 2271.0 | 2094.2 | 2197.3 |
| 10 mg phenylephrine hydrochloride + 500 mg paracetamol (n = 30) | 0.7 | 3947,0 | 2156.7 | 2245.8 |
| **Ratio** | **ND** | **134%** | **103%** | **102%** |

In a third cross-over study, 6 adult human subjects received either two tablets of *Maxiclear*^{™} PE 2.5 (500 mg paracetamol plus 2.5 mg phenylephrine hydrochloride) to deliver 1 ,000 mg paracetamol plus 5 mg phenylephrine hydrochloride, or one tablet of *Sudafed*^{™} PE (10 mg phenylephrine hydrochloride). It was observed that with the combination the 5 mg phenylephrine dose produced a plasma time-concentration curve similar to that for 10 mg phenylephrine administered alone. Pharmacokinetic parameters are summarised in Table 4 below.

**Table 4**

| **Pharmacokinetic Results (untransformed data) of Patients From the Third Study** | | | | |
|---|---|---|---|---|
| | **Tmax* (h)** | **Cmax (pg/mL)** | **AUC0n (h*pg/mL)** | **AUCtot (h*pg/mL)** |
| **Mean (SD)** | | | | |
| 5 mg phenylephrine hydrochloride + 1,000 mg paracetamol | 0.8 | 1597.9 | 1598.7 | 1842.1 |
| 10 mg phenylephrine hydrochloride (n = 6) | 0.7 | 1131.9 | 1705.2 | 1915.5 |
| Ratio | ND | 165.5 | 95.6 | 94.9 |

Assessing the effect of paracetamol in different doses on phenylephrine hydrochloride absorption into the body measured by the area under the plasma concentration over time (AUCtot) curve, it can be seen that the ratio was increased by 1.98-2.09 due to 1,000 mg paracetamol. The slight difference observed is thought primarily to be due to minor differences in formulations. For lower doses of paracetamol (500 mg) the AUCtot is increased by a factor of 1.60. This is consistent with lower amounts of paracetamol being present to compete with phenylephrine hydrochloride for absorption. Based on a linear relationship over the 500-1, 000mg dose range, the increase of the phenylephrine hydrochloride AUCtot for a paracetamol 650 mg dose is estimated to be approximtely 1.73.

**Table 5**

| **Effect of Different Doses of Paracetamol on Phenylephrine [PE] Absorption** | | |
|---|---|---|
| **Treatment (amount paracetamol)** | **AUCtot (h*pg/mL)** | **Ratio** |
| *Maxigesic*^{™} PE (1,000 mg paracetamol + 5 mg phenylephrine hydrochloride + 300 mg ibuprofen | 2311.4 | 2.09 |
| *Sudafed*^{™} PE Sinus & Pain Relief (1,000 mg paracetamol + 10 mg phenylephrine hydrochloride) | 2192.0 | 1.98 |
| Calculation for Paracetamol 650 mg (650 mg paracetamol + 10 mg phenylephrine hydrochloride) | | 1.73 |
| *Panadol*^{™} + *Sudafed*^{™} PE Nasal Decongestant (500 mg paracetamol + 10 mg phenylephrine hydrochloride) | 1779.8 | 1.60 |
| 10 mg phenylephrine hydrochloride (no paracetamol) | 1104.6 | 1.00 |

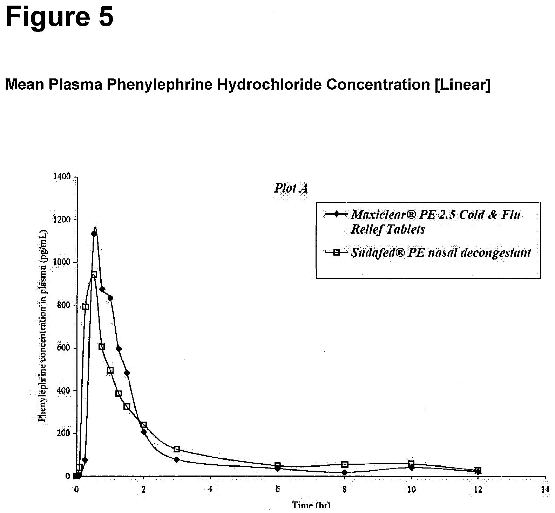

Figure 5 is a graph for the clinical data obtained indicating that 2 tablets of 2.5 mg phenylephrine hydrochloride + 500 mg paracetamol (to give a total of 5 mg phenylephrine hydrochloride + 1 ,000 mg paracetamol) is comparable to 10 mg phenylephrine hydrochloride alone.

While some preferred embodiments of the invention has been described by way of example it should be appreciated that modifications and improvements can occur without departing from the scope of the following claims.

## Claims

1. A combination medicament for use in the treatment of upper respiratory mucosal congestion in tablet, capsule, powder or liquid form,
**characterised in that** the medicament has phenylephrine hydrochloride and paracetamol in proportions suitable for, and the medicament is for, providing an adult with
a) 4 mg - 7.5 mg phenylephrine hydrochloride in combination with 950 mg - 1000 mg paracetamol, or
b) 5 mg - 7.5 mg phenylephrine hydrochloride in combination with 600 mg - 700 mg paracetamol.

2. A combination medicament for use according to claim 1, for providing an adult with a combination of 5 mg to 6.5 mg phenylephrine hydrochloride and 950 mg - 1,000 mg paracetamol.

3. A combination medicament for use according to claim 1, for providing an adult with a combination of 5 mg to 6.2 mg phenylephrine hydrochloride and 950 mg - 1,000 mg paracetamol.

4. A combination medicament for use according to claim 1, for providing an adult with a combination of 5 mg phenylephrine hydrochloride and 950 mg - 1000 mg paracetamol.

5. A combination medicament for use according to claim 1 for providing an adult with a combination of 5.8 mg to 7.1 mg phenylephrine hydrochloride and 625 mg - 675 mg paracetamol.

6. A combination medicament for use according to claim 1, for providing an adult with a combination of 5.8 mg phenylephrine hydrochloride and 625 mg - 675 mg paracetamol.

7. A combination medicament for use according to claim 1, where the medicament of part a) or part b) is produced in dosage units containing both named active ingredients for two dosage units to provide the doses mentioned in part a) or part b).

8. A combination medicament for use according to claim 1, where the medicament is produced in dosage units containing both named active ingredients sufficient for two dosage units to provide the doses mentioned in any one of claims 2 to 6 respectively.

9. A medicament for use according to claim 1, for providing an adult with 5 mg phenylephrine hydrochloride in combination with 1,000 mg paracetamol.

10. A medicament for use according to claim 1, for providing an adult with 6 mg phenylephrine hydrochloride in combination with 650 mg paracetamol.

## Patentansprüche

1. Ein Kombinationsmedikament zur Anwendung bei der Behandlung von Schleimhautstauungen der oberen Atemwege in Tabletten-, Kapsel-, Pulver- oder Flüssigform,
**dadurch gekennzeichnet, dass** das Medikament, geeignet, um einem Erwachsenen verabreicht zu werden, Phenylephrinhydrochlorid und Paracetamol in folgenden Anteilen enthält:
a) 4 mg bis 7,5 mg Phenylephrinhydrochlorid in Kombination mit 950 mg bis 1000 mg Paracetamol, oder
b) 5 mg bis 7,5 mg Phenylephrinhydrochlorid in Kombination mit 600 mg bis 700 mg Paracetamol.

2. Ein Kombinationsmedikament zur Anwendung nach Anspruch 1, zum Verabreichen einer Kombination aus 5 mg bis 6,5 mg Phenylephrinhydrochlorid und 950 mg bis 1.000 mg Paracetamol an einen Erwachsenen.

3. Ein Kombinationsmedikament zur Anwendung nach Anspruch 1, zum Verabreichen einer Kombination aus 5 mg bis 6,2 mg Phenylephrinhydrochlorid und 950 mg bis 1.000 mg Paracetamol an einen Erwachsenen.

4. Ein Kombinationsmedikament zur Anwendung nach Anspruch 1, zum Verabreichen einer Kombination aus 5 mg Phenylephrinhydrochlorid und 950 mg bis 1000 mg Paracetamol an einen Erwachsenen.

5. Ein Kombinationsmedikament zur Anwendung nach Anspruch 1 zum Verabreichen einer Kombination aus 5,8 mg bis 7,1 mg Phenylephrinhydrochlorid und 625 mg bis 675 mg Paracetamol an einen Erwachsenen.

6. Ein Kombinationsmedikament zur Anwendung nach Anspruch 1, zum Verabreichen einer Kombination aus 5,8 mg Phenylephrinhydrochlorid und 625 mg bis 675 mg Paracetamol an einen Erwachsenen.

7. Ein Kombinationsmedikament zur Anwendung nach Anspruch 1, wobei das Medikament gemäß Abschnitt a) oder Abschnitt b) in Dosierungseinheiten hergestellt wird, die beide genannten Wirkstoffe für zwei Dosierungseinheiten enthalten, um die in Abschnitt a) oder Abschnitt b) angegebenen Dosen bereitzustellen.

8. Ein Kombinationsmedikament zur Anwendung nach Anspruch 1, wobei das Medikament in Dosierungseinheiten hergestellt wird, die beide genannten Wirkstoffe in einer Menge enthalten, die für zwei Dosierungseinheiten ausreicht, um die in einem der Ansprüche 2 bis 6 genannten Dosen bereitzustellen.

9. Ein Medikament zur Anwendung nach Anspruch 1, zum Verabreichen von 5 mg Phenylephrinhydrochlorid in Kombination mit 1.000 mg Paracetamol an einen Erwachsenen.

10. Ein Medikament zur Anwendung nach Anspruch 1, zum Verabreichen von 6 mg Phenylephrinhydrochlorid in Kombination mit 650 mg Paracetamol an einen Erwachsenen.

## Revendications

1. Médicament combiné destiné à une utilisation pour le traitement de la congestion des muqueuses respiratoires supérieures sous la forme de comprimé, de capsule, de poudre ou de liquide,
**caractérisé en ce que** le médicament comprend du chlorhydrate de phényléphrine et du paracétamol dans les proportions adaptées à un adulte et le médicament est destiné à lui délivrer :
a) de 4 mg à 7,5 mg de chlorhydrate de phényléphrine en association avec de 950 mg à 1000 mg de paracétamol, ou
b) de 5 mg à 7,5 mg de chlorhydrate de phényléphrine en association avec de 600 mg à 700 mg de paracétamol.

2. Médicament combiné destiné à une utilisation selon la revendication 1, afin de délivrer à un adulte un mélange de 5 mg à 6,5 mg de chlorhydrate de phényléphrine et de 950 mg à 1000 mg de paracétamol.

3. Médicament combiné destiné à une utilisation selon la revendication 1, afin de délivrer à un adulte un mélange de 5 mg à 6,2 mg de chlorhydrate de phényléphrine et de 950 mg à 1000 mg de paracétamol.

4. Médicament combiné destiné à une utilisation selon la revendication 1, afin de délivrer à un adulte un mélange de 5 mg de chlorhydrate de phényléphrine et de 950 mg à 1000 mg de paracétamol.

5. Médicament combiné destiné à une utilisation selon la revendication 1, afin de délivrer à un adulte un mélange de 5,8 mg à 7,1 mg de chlorhydrate de phényléphrine et de 625 mg à 675 mg de paracétamol.

6. Médicament combiné destiné à une utilisation selon la revendication 1, afin de délivrer à un adulte un mélange 5,8 mg de chlorhydrate de phényléphrine et 625 mg à 675 mg de paracétamol.

7. Médicament combiné destiné à une utilisation selon la revendication 1, dans lequel le médicament de la partie a) ou le médicament de la partie b) est produit dans des unités de dosage contenant les deux substances actives citées afin que les deux unités de dosage délivrent les doses mentionnées dans la partie a) ou la partie b).

8. Médicament combiné destiné à une utilisation selon la revendication 1, dans lequel le médicament est produit dans des unités de dosage contenant les deux substances actives citées en quantité suffisante afin que les deux unités de dosage délivrent respectivement les doses mentionnées dans l'une quelconque des revendications 2 à 6.

9. Médicament destiné à une utilisation selon la revendication 1, afin d'administrer à un adulte 5 mg de chlorhydrate de phényléphrine en association avec 1000 mg de paracétamol.

10. Médicament destiné à une utilisation selon la revendication 1, afin d'administrer à un adulte 6 mg de chlorhydrate de phényléphrine en association avec 650 mg de paracétamol.
